(19) Europäisches Patentamt
European Patent Office
Office européen des brevets

(11) Veröffentlichungsnummer : **0 013 894**
**B1**

(12) **EUROPÄISCHE PATENTSCHRIFT**

(45) Veröffentlichungstag der Patentschrift : **03.02.82**

(21) Anmeldenummer : **80100071.2**

(22) Anmeldetag : **08.01.80**

(51) Int. Cl.³ : **C 07 D401/12**, C 07 D405/12, A 61 K 31/37, A 61 K 31/47

(54) **Neue Arylether, Verfahren zu deren Herstellung sowie diese Verbindungen enthaltende Arzneimittel.**

(30) Priorität : **15.01.79 DE 2901336**

(43) Veröffentlichungstag der Anmeldung : **06.08.80 (Patentblatt 80/16)**

(45) Bekanntmachung des Hinweises auf die Patenterteilung : **03.02.82 Patentblatt 82/05**

(84) Benannte Vertragsstaaten : **AT BE CH DE FR GB IT LU NL SE**

(56) Entgegenhaltungen :
**FR - A - 2 118 191**
**FR - A - 2 344 538**
**GB - A - 1 410 783**

(73) Patentinhaber : **BOEHRINGER MANNHEIM GMBH**
**Sandhofer Strasse 112-132 Postfach 31 01 20**
**D-6800 Mannheim 31-Waldhof (DE)**

(72) Erfinder : **Friebe, Walter-Gunar, Dr. rer. nat.**
**Heidelberger Landstrasse 111d**
**D-6100 Darmstadt 13 (DE)**
Erfinder : **Winter, Werner, Dr. rer. nat.**
**Mannheimerstrasse 9**
**D-6148 Heppenheim (DE)**
Erfinder : **Thiel, Max, Dr. rer. nat.**
**S 6, 35**
**D-6800 Mannheim (DE)**
Erfinder : **Roesch, Androniki, Dr. Med.**
**Am Oberen Luisenpark 22**
**D-6800 Mannheim 1 (DE)**
Erfinder : **Wilhelms, Otto-Henning, Dr. rer. nar.**
**Odenwaldstrasse 25/2**
**D-6941 Weinheim-Rittenweier (DE)**

(74) Vertreter : **Weber, Manfred (DE)**
**Fa. Boehringer Mannheim GmbH Postfach 310120**
**D-6800 Mannheim 31 (DE)**

EP 0 013 894 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Neue Arylether, Verfahren zu deren Herstellung sowie diese Verbindungen enthaltende Arzneimittel

Die Erfindung betrifft Arylether-Derivate der allgemeinen Formel I

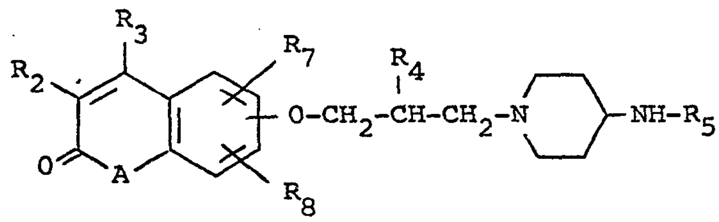

(I)

in der

A ein Sauerstoffatom oder die Gruppe N-$R_1$ darstellt,

wobei

$R_1$ ein Wasserstoffatom oder ein niederer Alkylrest sein kann,

$R_2$ und $R_3$, die gleich oder verschieden sein können, jeweils ein Wasserstoffatom oder einen niederen Alkylrest,

$R_4$ ein Wasserstoffatom oder die Hydroxyl-Gruppe,

$R_5$ ein Wasserstoffatom oder eine Niederalkanoyl-Gruppe, die ein- oder mehrfach durch Halogen, durch Phenyl, oder durch Benzthiazol-Derivate substituiert sein kann, oder eine gegebenenfalls zweifach durch Methoxy substituierte Cinnamoyl-Gruppe, oder eine Benzoyl-Gruppe, die ein- oder mehrfach durch Halogen, Hydroxyl, Niederalkyl, Niederalkoxy, Carboxyl, Nitro, Amino, Nitril, Trifluormethyl, Carbamoyl oder Benzyl substituiert sein kann, oder eine Furancarbonyl-, Thiophencarbonyl-, oder Pyridincarbonyl-Gruppe, oder eine $C_3$-$C_7$-Cycloalkylcarbonyl-Gruppe oder eine Benzolsulfonyl- oder Methansulfonyl-Gruppe,

$R_7$ ein Wasserstoffatom, einen niederen Alkylrest oder einen niederen Alkanoylrest und

$R_8$ ein Wasserstoffatom, einen niederen Alkylrest, einen niederen Alkoxyrest oder die Hydroxylgruppe bedeuten, sowie deren pharmakologisch verträgliche Salze, Verfahren zu deren Herstellung sowie ihre Verwendung bei der Bekämpfung von allergischen Krankheiten.

Weiterhin betrifft die Erfindung pharmazeutische Präparate mit einem Gehalt an Verbindungen der allgemeinen Formel I sowie die Verwendung von Verbindungen der allgemeinen Formel I zur Herstellung solcher Präparate, wobei die erfindungsgemäßen Substanzen gegebenenfalls in Form von Salzen nichttoxischer anorganischer oder organischer Säuren Anwendung finden.

Die niederen Alkyl-Gruppen der Substituenten $R_1$, $R_2$, $R_3$, $R_7$ und $R_8$ können geradkettig und verzweigt sein und enthalten 1-6, vorzugsweise 1-4 Kohlenstoffatome.

Eine niedere Alkoxy-Gruppe des Substituenten $R_8$ enthält 1-6, vorzugsweise 1-4 Kohlenstoffatome. Eine niedere Alkanoyl-Gruppe des Substituenten $R_7$ enthält 1-6 Kohlenstoffatome, vorzugsweise versteht man hierunter die Acetyl-Gruppe.

Die Acyl-Gruppen des Substituenten $R_5$ bedeuten Niederalkanoyl-Gruppen, beispielsweise die Acetyl-Gruppe, die gegebenenfalls ein- oder mehrfach durch Halogen, durch Phenyl, oder durch Benzthiazol-Derivate, substituiert sind, oder eine gegebenenfalls zweifach durch Methoxy substituierte Cinnamoyl-Gruppe, oder eine Benzoyl-Gruppe die ein- oder mehrfach durch Halogen, Hydroxyl, Niederalkyl, Niederalkoxy Carboxyl, Nitro, Amino, Nitril, Trifluormethyl, Carbamoyl oder Benzyl substituiert sein kann. Die Niederalkyl-Reste in den genannten Gruppen enthalten jeweils 1 bis 6, vorzugsweise 1 bis 4 Kohlenstoffatome.

Des weiteren bedeutet $R_5$ einen Furancarbonyl-, Thiophencarbonyl-, Pyridin-carbonyl-Rest, eine $C_3$-$C_7$-Cycloalkylcarbonyl-Gruppe oder eine Benzolsulfonyl- oder Methansulfonyl-Gruppe.

Als Halogenatome kommen Fluor, Chlor und Brom in Frage.

Ausser den in den Beispielen genannten Verbindungen sind Gegenstand der Erfindung alle Substanzen, die jede moegliche Kombination der in den Beispielen genannten Substituenten aufweisen.

In der DE-OS 19 32 384 und DE-OS 21 23 924 sind Piperazine beschrieben, die über eine Oxypropyl-Gruppe mit einem 2-Oxo-1,2-dihydro-chinolin-bzw. einem Cumarin-Rest verbunden sind, wobei die Chinoline eine ausgeprägte coronargefäßerweiternde Wirkung aufweisen. Es wurde nun gefunden, daß Chinoline und Cumarine, die über eine Oxyalkyl-Gruppe mit einem Piperidin-Ring verbunden sind, eine ausgeprägte antiallergische Wirkung aufweisen, wie sie im pharmakologischen Test der passiven cutanen Anaphylaxie (PCA-Test) in vivo bei Ratten nachgewiesen werden kann. So wurde gefunden, daß von dem Handelsprodukt Diethylcarbamazin (1-Diethylcarbamazoyl-4-methyl-piperazin) ca. vierzigmal mehr Substanz eingesetzt werden muß, als von der Verbindung 7-[3-(4-Benzamido-piperidin)-propoxy]-3,4-dimethyl-cumarin, um eine vergleichbare Inhibierung der passiven cutanen anaphylactoiden Reaktion zu erreichen. Außerdem zeigen sie einen starken Anti-histamineffekt. Die erfindungsgemäßen Verbindungen der allgemeinen Formel I können daher besonders vorteilhaft bei der Bekämpfung von allergischen Krankheiten, beispielsweise von allergischem Asthma, Heuschnupfen und Urticaria, verwendet werden.

Die neuen Verbindungen der allgemeinen Formel I lassen sich auf verschiedene Weise zu Substanzen weiterverarbeiten, die ebenfalls pharmakologische Wirksamkeit, insbesondere antiallergische oder antihypertensive Wirksamkeit, aufweisen. Sie stellen daher auch wertvolle Zwischenprodukte zur Herstellung von pharmakologisch wirksamen Stoffen dar.

Das erfindungsgemaesse Verfahren zur Herstellung der Verbindungen der allgemeinen Formel I ist dadurch gekennzeichnet, dass man in an sich bekannter Weise.

eine Verbindung der allgemeinen Formel II

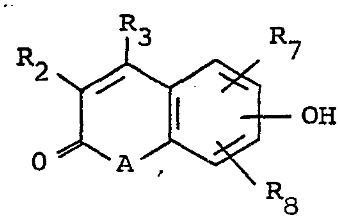

(II)

in der

A, $R_2$, $R_3$, $R_7$ und $R_8$ die oben angegebene Bedeutung haben, mit einer Verbindung der allgemeinen Formel III

$$X - CH_2 - \overset{\overset{R_6}{|}}{CH} - CH_2 - Y \qquad (III)$$

in der

X und Y reaktive Reste bedeuten und $R_6$ ein Wasserstoffatom, die Hydroxyl-Gruppe oder auch zusammen mit Y ein Sauerstoffatom sein kann, und einer Verbindung der allgemeinen Formel IV

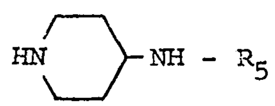

(IV)

in der

$R_5$ die obengenannte Bedeutung hat, umsetzt, anschliessend gewuenschtenfalls die Gruppe $R_5$ durch bekannte Verfahren in eine andere Gruppe $R_5$ umwandelt, für den Fall, daß $R_1$ ein Wasserstoffatom bedeutet, gegebenenfalls nachträglich N-alkyliert und das so erhaltene Reaktionsprodukt gewünschtenfalls in ein pharmakologisch verträgliches Salz überführt.

Beispielsweise kann eine Verbindung der allgemeinen Formel I mit einem bestimmten Substituenten $R_5$ durch Verseifen und nachfolgende Acylierung mit einer Verbindung $R_5$-Z, wobei Z einen reaktiven Rest und $R_5$ eine andere, durch den Anspruch umfaßte Gruppe bedeutet, in eine Verbindung der allgemeinen Formel I mit einem anderen Substituenten $R_5$ umgewandelt werden. Ferner läßt sich in bekannter Weise eine Verbindung der allgemeinen Formel I, in der der Substituent $R_5$ eine Nitro-Gruppe enthält, durch Reduktion in eine Verbindung der allgemeinen Formel I mit einer Amino-Gruppe im $R_5$-Substituenten überführen.

Als reaktive Reste X und Y der Verbindungen der allgemeinen Formel III kommen alle üblichen Gruppen infrage, die sich nucleophil verdrängen lassen. Besonders bevorzugt sind Chlor und Brom sowie die Mesyloxy- und Tosyl-oxy-Gruppe.

Reaktive Reste Z koennen alle Reste sein, die in der Peptid-Chemie zur Aktivierung von Carbonsaeuren Verwendung finden, beispielsweise Halogenatome, die Azido-Gruppe, Alkyloxy-, Aryloxy- und Acyloxy-Gruppen.

Das erfindungsgemäße Verfahren führt man vorzugsweise so durch, daß man zunächst eine Verbindung der allgemeinen Formel III mit einer Verbindung der allgemeinen Formel II kondensiert und das erhaltene Reaktionsprodukt isoliert. Dieses Zwischenprodukt wird dann mit einer Verbindung der allgemeinen Formel IV zur Reaktion gebracht. Die erstgenannte Reaktion erfolgt zweckmäßig in alkalischem Medium in einem geeigneten Lösungsmittel wei beispielsweise einem niederen Alkohol, beispielsweise Ethanol oder Isopropanol, in Gegenwart von einem Natriumalkoholat, aber auch von Tetrahydrofuran, Methoxyethanol, Ethoxyethanol, Dimethylformamid, Dimethylsulfoxid oder Hexametapol, wobei als Base auch Alkali- und Erdalkalimetallhydroxide oder -hydride Verwendung finden können.

Die Reaktion des vorstehend genannten Zwischenproduktes mit einer Verbindung der allgemeinen Formel IV kann in den vorgenannten Loesungsmitteln erfolgen, als Basen lassen sich auch tertiaere Amine wie Triethylamin, eine Huenig-Base, ein stark basischer Ionenaustauscher oder ein Ueberschuss der Verbindung der allgemeinen Formel IV verwenden.

Eine andere Variante besteht darin, zunaechst eine Verbindung der allgemeinen Formel III mit einer Verbindung der allgemeinen Formel IV zur Reaktion zu bringen ; anschliessend wird das erhaltene Reaktionsgemisch mit einer Verbindung der allgemeinen Formel II zum gewuenschten Endprodukt der allgemeinen Formel I umgesetzt. Die Startreaktion erfolgt in den vorstehend genannten Loesungsmitteln, vorteilhafterweise unter Zugabe eines tertiaeren Amins, wie z.B. Triethylamin, einer Huenig-Base oder eines stark basischen Ionenaustauschers, aber auch beispielsweise in Gegenwart von Kalium-t-butoxid in Dimethylsulfoxid.

Eine nachtraegliche Umwandlung der Gruppe $R_5$ in der allgemeinen Formel I in eine andere Gruppe $R_5$ erfolgt beispielsweise als Austausch eines Acylrestes $R_5$ gegen einen anderen Rest $R_5$. Hierzu werden Verbindungen der Formel I zunaechst in saurem oder alkalischem Milieu verseift und die erhaltenen Zwischenprodukte vorzugsweise in Gegenwart eines saeurebindenden Mittels nach bekannten Methoden acyliert.

Die vorgenannten Zwischenprodukte der Formel I, in denen $R_5$ ein Wasserstoffatom bedeutet, können aber auch so hergestellt werden, daß man eine Verbindung der allgemeinen Formel II mit einer Verbindung der allgemeinen Formel III und 4-Oximinopiperidin umsetzt und das erhaltene Kondensationsprodukt anschließend reduziert oder hydriert.

Desweiteren kann in Verbindungen der Formel I, in denen der Acylrest $R_5$ eine Nitro-Gruppe enthält, die Nitro-Gruppe durch bekannte Methoden in eine Amino-Gruppe umgewandelt werden, z.B. durch katalytische Hydrierung.

Die Ausgangsverbindungen der allgemeinen Formeln II, III und IV sind literaturbekannte Substanzen oder koennen in Analogie zu literaturbekannten Verfahren hergestellt werden.

Die pharmakologisch vertraeglichen Salze erhaelt man in ueblicher Weise, z.B. durch Neutralisation der Verbindungen der Formel I mit nichttoxischen anorganischen oder organischen Saeuren wie z.B. Salzsaeure, Schwefelsaeure, Phosphorsaeure, Bromwasserstoffsaeure, Essigsaeure, Milchsaeure, Zitronensaeure, Aepfelsaeure, Salicyclsaeure, Malonsaeure, Maleinsaeure oder Bernsteinsaeure.

Zur Herstellung von Arzneimitteln werden die Verbindungen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methyl-cellulose, Talkum, hochdisperse Kieselsäure, hoehermolekulare Polymere (wie Polyethylenglykole).

Für die orale Applikation geeignete Zubereitungen können gewünschtenfalls Geschmacks- und Süßstoffe enthalten. Für die aeusserliche Anwendung koennen die erfindungsgemaessen Substanzen I auch in Form von Pudern und Salben verwendet werden, sie werden dazu z.B. mit pulverfoermigen, physiologisch vertraeglichen Verduennungsmitteln bzw. ueblichen Salbengrundlagen vermischt.

Die verabreichte Dosierung haengt vom Alter, der Gesundheit und dem Gewicht des Empfaengers, dem Ausmass der Krankheit, der Art gleichzeitiger gegebenenfalls durchgefuehrter weiterer Behandlungen, der Haeufigkeit der Behandlungen und der Art der gewuenschten Wirkung ab. Ueblicherweise betraegt die taegliche Dosis der aktiven Verbindung 0.1 bis 50 mg/kg Koerpergewicht. Normalerweise sind 0.5 bis 40 und vorzugsweise 1.0 bis 20 mg/kg/Tag in einer oder mehreren Anwendungen pro Tag wirksam, um die gewuenschten Resultate zu erhalten.

Ausser den in den nachstehenden Beispielen genannten Substanzen werden die folgenden Verbindungen bevorzugt :

6-[3-(4-Benzamido-piperidino)-propoxy]-1-n-butyl-4-methyl-2-oxo-1,2-dihydro-chinolin

6-[3-(4-Benzamido-piperidino)-propoxy]-4-methyl-1-(2-methyl-butyl)-2-oxo-1,2-dihydro-chinolin.

Beispiel 1

7-[3-(4-Benzamido-piperidino)-propoxy]-3,4-dimethyl-cumarin

Eine Mischung aus 9.34 g (0.03 mol) 7-(3-Brom-propoxy)-3,4-dimethyl-cumarin, 6.13 g (0.03 mol) 4-Benzamido-piperidin, 12.43 g (0.09 mol) Triethylamin und 100 ml Tetrahydrofuran wird 6 Stunden unter Rueckfluss erhitzt, im Vakuum eingeengt, der Rueckstand in Wasser aufgenommen, mit Methylenchlorid extrahiert und der Extrakt eingeengt. Nach Umkristallisation aus Methanol erhaelt man 6.5 g 7-[3-(4-Benzamido-piperidino)-propoxy]-3,4-dimethyl-cumarin (50 % d. Th.) vom Schmp. 201-203 °C.

Beispiel 2

In analoger Weise wie in Beispiel 1 beschrieben erhaelt man :

| | Bezeichnung | Ausbeute % | Schmelzpunkt °C (Lösungsmittel) |
|---|---|---|---|
| a) | 3,4-Dimethyl-7-(3-[4-(4-fluor-benzamido)-piperidino]-propoxy)-cumarin aus 7-(3-Brom-propoxy)-3,4-dimethyl-cumarin und 4-(4-Fluor-benzamido)-piperidin | 59 | Hydrochlorid 268-270 (Isopropanol) |
| b) | 3,4-Dimethyl-7-(3-[4-(4-methoxy-benzamido)-piperidino]-propoxy)-cumarin aus 7-(3-Brom-propoxy)-3,4-dimethyl-cumarin und 4-(4-Methoxy-benzamido)-piperidin | 43 | Hydrochlorid 266-268 (Isopropanol) |
| c) | 3,4-Dimethyl-7-(3-[4-(4-methyl-benzamido)-piperidino]-propoxy)-cumarin aus 7-(3-Brom-propoxy)-3,4-dimethyl-cumarin und 4-(4-Methyl-benzamido)-piperidin | 38 | Hydrochlorid 272-274 (Isopropanol) |
| d) | 7-(3-[4-(4-t-Butyl-benzamido)-piperidino]-propoxy)-3,4-dimethyl-cumarin aus 7-(3-Brom-propoxy)-3,4-dimethyl-cumarin und 4-(4-t-Butyl-benzamido)-piperidin | 61 | Hydrochlorid 295-296 (Isopropanol) |
| e) | 7-[3-(4-Acetamido-piperidino)-propoxy]-3,4-dimethyl-cumarin aus 7-(3-Brom-propoxy)-3,4-dimethyl-cumarin und 4-Acetamido-piperidin | 28 | Hydrochlorid 172-174 (Isopropanol |
| f) | 3,4-Dimethyl-7-[3-(4-phenylacetamido-piperidino)-propoxy]-cumarin aus 7-(3-Brom-propoxy)-3,4-dimethyl-cumarin und 4-Phenylacetamido-piperidin | 49 | 138-140 (Essigester) |
| g) | 3,4-Dimethyl-7-(3-[4-(2-methyl-propion-amido)-piperidino]-propoxy)-cumarin aus 7-(3-Brom-propoxy)-3,4-dimethyl-cumarin und 4-(2-Methyl-propionamido)-piperidin | 91 | 176-178 (Isopropanol) |
| h) | 7-[3-(4-Cyclohexancarbonamido-piperidino)-propoxy]-3,4-dimethyl-cumarin aus 7-(3-Brom-propoxy)-3,4-dimethyl-cumarin und 4-Cyclohexancarbonamido-piperidin | 36 | 174-177 (Isopropanol) |

## Beispiel 3

7-[3-(4-Benzamido-piperidino)-propoxy]-cumarin

Zu der Loesung von 0.58 g (0.025 mol) Natrium in 100 ml Isopropanol gibt man 4.65 g (0.025 mol) 7-Hydroxy-cumarin, erhitzt 10 Minuten unter Rueckfluss, kuehlt ab und fuegt 7.25 g (0.025 mol) 3-(4-Benzamido-piperidino)-propylchlorid in 25 ml Isopropanol zu. Nach 6 Stunden Rueckfluss engt man im Vakuum ein, nimmt in Methylenchlorid auf, waescht mit 2 N Natronlauge und anschliessend mit Wasser, engt ein und kristallisiert aus Ethanol um.

Man erhaelt 8.9 g 7-[3-(4-Benzamido-piperidino)-propoxy]-cumarin (88 % d. Th.) von Schmp. 157-158 °C.

Beispiel 4

In analoger Weise wie in Beispiel 3 beschrieben erhaelt man :

| | | | |
|---|---|---|---|
| a) | 6-[3-(4-Benzamido-piperidino)-propoxy]-4-methyl-cumarin aus 6-Hydroxy-4-methyl-cumarin und 3-(4-Benzamido-piperidino)-propylchlorid | 66 | 184-186 (Methanol) |
| b) | 7-[3-(4-Benzamido-piperidino)-propoxy]-4-methyl-cumarin aus 7-Hydroxy-4-methyl-cumarin und 3-(4-Benzamido-piperidino)-propylchlorid | 48 | 164-166 (Isopropanol) |

Beispiel 5

1-(4-Benzamido-piperidino)-3-(3,4-dimethyl-cumarin-7-yloxy)-2-propanol

Eine Mischung von 8.1 g (0.033 mol) 3,4-Dimethyl-7-(2,3-epoxy-propoxy)-cumarin, 6.73 g (0.033 mol) 4-Benzamido-piperidin und 75 ml Ethanol wird 6 Stunden unter Rueckfluss erhitzt, eingeengt, der Rueckstand in Essigester aufgenommen, mit Wasser gewaschen, eingeengt und aus Essigester umkristallisiert.

Man erhaelt 2.8 g 1-(4-Benzamido-piperidino)-3-(3,4-dimethyl-cumarin-7-yloxy)-2-propanol (26 % d. Th.) vom Schmp. 178-180 °C.

Beispiel 6

7-(3-[4-(4-Chlor-benzamido)-piperidino]-propoxy)-3,4-dimethyl-cumarin

Zu der Mischung von 6.6 g (0.02 mol) 7-[3-(4-Amino-piperidino)-propoxy]-3,4-dimethyl-cumarin, 100 ml 2 N Natronlauge und 75 ml Tetrahydrofuran tropft man die Lösung von 3.85 g (0.022 mol) 4-Chlor-benzoylchlorid in 25 ml Tetrahydrofuran, rührt 5 Studen bei Raumtemperatur, filtriert und kristallisiert den Niederschlag aus Ethanol um. Man erhält 5.5 g 7-(3-[4-(4-Chlor-benzamido)-piperidino]-propoxy)-3,4-dimethyl-cumarin (59 % d.Th.) vom Schmp. 212-214 °C.

Das als Ausgangsstoff verwendete 7-[3-(4-Amino-piperidino)-propoxy]-3,4-dimethyl-cumarin kann auf folgende Weise erhalten werden :

Durch Umsetzung von 7-(3-Brom-propoxy)-3,4-dimethyl-cumarin und 4-Oximino-piperidin-hydrochlorid in dioxanischer Loesung in Gegenwart von Huenig-Base erhaelt man 3,4-Dimethyl-7-[3-(4-oximino-piperidino)-propoxy]-cumarin vom Schmp. 198-200 °C, woraus durch katalytische Hydrierung ueber Raney-Nickel in ammoniakalischem Methanol 7-[3-(4-Amino-piperidino)-propoxy]-3,4-dimethyl-cumarin vom Schmp. 92-94 °C entsteht.

Zu derselben Verbindung kommt man beispielsweise durch Behandlung von 7-[3-(4-Benzamido-piperidino)-propoxy]-3,4-dimethyl-cumarin mit alkoholischer Kalilauge.

Außerdem kann die Verbindung durch Umsetzung von 4-Amino-piperidin mit 7-(3-Brom-propoxy)-3,4-dimethyl-cumarin unter Rückfluß in Ethanol in Gegenwart von Triethylamin erhalten werden.

Beispiel 7

In analoger Weise wie in Beispiel 6 beschrieben erhaelt man :

| | Bezeichnung | Ausbeute % | Schmelzpunkt °C (Lösungsmittel) |
|---|---|---|---|
| a) | 3,4-Dimethyl-7-(3-[4-(2-hydroxy-benzamido)-piperidino]-propoxy)-cumarin aus 7-[3-(4-Amino-piperidino)-propoxy]-3,4-dimethyl-cumarin und Salicylsaeurechlorid | 67 | 192-194 (Essigester) |

| | Bezeichnung | Ausbeute % | Schmelzpunkt °C (Lösungsmittel) |
|---|---|---|---|
| b) | 3,4-Dimethyl-7-(3-[4-(thiophen-2-carbonamido)-piperidino]-propoxy)-cumarin<br>aus<br>7-[3-(4-Amino-piperidino)-propoxy]-3,4-dimethyl-cumarin und Thiophen-2-carbonsaeurechlorid | 31 | 197-200 (Isopropanol) |
| c) | 7-[3-(4-Benzolsulfonamido-piperidino)-propoxy]-3,4-dimethyl-cumarin<br>aus<br>7-[3-(4-Amino-piperidino)-propoxy]-3,4-dimethyl-cumarin und Benzolsulfonsaeurechlorid | 29 | 136-138 (Isopropanol) |
| d) | 7-(3-[4-(3,4-Dimethoxy-cinnamoylamido)-piperidino]-propoxy)-3,4-dimethyl-cumarin<br>aus<br>7-[3-(4-Amino-piperidino)-propoxy]-3,4-dimethyl-cumarin und 3,4-Dimethoxy-zimtsaeurechlorid | 37 | 195-196 (Isopropanol) |
| e) | 7-[3-(4-Cyclopropancarbonylamido-piperidino)-propoxy]-3,4-dimethyl-cumarin<br>aus<br>7-[3-(4-Amino-piperidino)-propoxy]-3,4-dimethyl-cumarin und Cyclopropancarbonsaeurechlorid | 33 | 190-191 (Essigester) |
| f) | 3,4-Dimethyl-7-(3-[4-(furan-2-carbonamido)-piperidino]-propoxy)-cumarin<br>aus<br>7-[3-(4-Amino-piperidino)-propoxy]-3,4-dimethyl-cumarin und Furan-2-carbonsäurechlorid | 40 | 182-183 (Methanol) |
| g) | 3,4-Dimethyl-7-(3-[4-(4-nitro-benzamido)-piperidino]-propoxy)-cumarin<br>aus<br>7-[3-(4-Amino-piperidino)-propoxy]-3,4-dimethyl-cumarin und 4-Nitro-benzoylchlorid | 62 | 210-212 (Methylen-chlorid) |
| h) | 7-(3-[4-(5-Chlor-2-methoxy-benzamido)-piperidino]-propoxy)-3,4-dimethyl-cumarin<br>aus<br>7-[3-(4-Amino-piperidino)-propoxy]-3,4-dimethyl-cumarin und 5-Chlor-2-methoxy-benzoylchlorid | 69 | 151-152 (Isopropanol) |
| i) | 7-(3-[4-(2,3-Dihydro-2-oxo-benzthiazol-3-yl-acetamido)-piperidino]-propoxy)-3,4-dimethyl-cumarin<br>aus<br>7-[3-(4-Amino-piperidino)-propoxy]-3,4-dimethyl-cumarin und 2,3-Dihydro-2-oxo-benzthiazol-3-yl-acetylchlorid | 58 | 221-222 (Methanol) |
| j) | 7-(3-[4-(5-Chlor-2,3-dihydro-2-oxo-benzthiazol-3-yl-acetamido)-piperidino]-propoxy)-3,4-dimethyl-cumarin<br>aus<br>7-[3-(4-Amino-piperidino)-propoxy]-3,4-dimethyl-cumarin und 5-Chlor-2,3-dihydro-2-oxo-benzthiazol-3-yl-acetyl-chlorid | 51 | 238-240 (Methanol) |

## Beispiel 8

6-[3-(4-Benzamido-piperidino)-propoxy]-4-methyl-2-oxo-1,2-dihydro-chinolin

4.9 g (0.03 mol) 6-Hydroxy-4-methyl-2-oxo-1,2-dihydro-chinolin werden in 60 ml Wasser und 30 ml 1 N Natronlauge geloest. Anschliessend engt man die Loesung weitgehend ein, nimmt den Rueckstand in

75 ml Dimethylformamid auf und versetzt mit 9.8 g (0.035 mol) 3-(4-Benzamido-piperidino)-propylchlorid. Nach vierstuendigem Ruehren bei 100 °C wird das Loesungsmittel im Vakuum entfernt und der Rueckstand in Methylenchlorid aufgenommen, getrocknet und eingeengt.

Nach Umkristallisation aus Ethanol erhaelt man 6.8 g 6-[3-(4-Benzamido-piperidino)-propoxy]-4-methyl-2-oxo-1,2-dihydro-chinolin (54 % d. Th.) vom Schmp. 243-245 °C.

Beispiel 9

7-[3-(4-Benzamido-piperidino)-propoxy]-4-methyl-2-oxo-1,2-dihydro-chinolin

In analoger Weise wie in Beispiel 8 beschrieben erhaelt man durch Umsetzung von 7-Hydroxy-4-methyl-2-oxo-1,2-dihydro-chinolin mit 3-(4-Benzamido-piperidino)-propylchlorid in Methoxyethanol die Titelverbindung von Schmp. 275-276 °C in einer Ausbeute von 46 % d. Th.

Beispiel 10

6-[3-(4-Benzamido-piperidino)-propoxy]-1,4-dimethyl-2-oxo-1,2-dihydro-chinolin

Zu der Loesung von 3.55 g (0.02 mol) 1,4-Dimethyl-6-hydroxy-2-oxo-1,2-dihydro-chinolin, 7,0 g (0.025 mol) 3-(4-Benzamido-piperidino)-propylchlorid und 3.0 g (0.02 mol) Natriumiodid in 10 ml Wasser und 50 ml Methanol tropft man unter Rueckfluss die Loesung von 0.8 g Natriumhydroxid in 10 ml Wasser, kocht 3 Stunden nach, engt ein, nimmt den Rueckstand in Methylen-chlorid auf, waescht mit verd. Natronlauge, engt ein und kristallisiert aus Isopropanol um. Man erhaelt 4.0 g Titelverbindung (47 % d. Th.) vom Schmp. 188-190 °C.

Beispiel 11

7-[3-(4-Benzamido-piperidino)-propoxy]-1,4-dimethyl-2-oxo-1,2-dihydro-chinolin

In analoger Weise wie in Beispiel 10 beschrieben erhaelt man durch Umsetzung von 1,4-Dimethyl-7-hydroxy-2-oxo-1,2-dihydro-chinolin mit 3-(4-Benzamido-piperidino)-propylchlorid in Methoxyethanol die Titelverbindung vom Schmp. 193-195 °C in einer Ausbeute von 67 % d. Th.

a) 7-[3-(4-Benzamido-piperidino)-propoxy]-1-ethyl-4-methyl-2-oxo-1,2-dihydro-chinolin

Entsprechend Beispiel 11 erhaelt man durch Umsetzung von 1-Ethyl-7-hydroxy-4-methyl-2-oxo-1,2-dihydro-chinolin mit 3-(4-Benzamido-piperidino)-propylchlorid die Titelverbindung vom Schmp. 164-165 °C (aus Dichlormethan/Ether) in einer Ausbeute von 59 % d. Th.

Beispiel 12

7-(3-[4-(4-Amino-benzamido)-piperidino]-propoxy)-3,4-dimethyl-cumarin

Eine Loesung von 4.2 g (0.009 mol) 3,4-Dimethyl-7-(3-[4-(4-nitro-benzamido)-piperidino]-propoxy)-cumarin in 100 ml Methanol und 100 ml Tetrahydrofuran wird bei Raumtemperatur und 1 bar Wasserstoffdruck ueber 1 ml Raney-Nickel hydriert. Man filtriert, waescht mit Methylenchlorid und Methanol nach, engt das Filtrat ein und kristallisiert den Rueckstand aus Methanol um. Man erhaelt 2.8 g Titelverbindung (71 % d. Th.) vom Schmp. 242-245 °C.

Beispiel 13

7-[3-(4-Benzamido-piperidino)-propoxy]-3-n-butyl-4-methyl-cumarin

4.65 g (0.02 mol) 7-Hydroxy-3-n-butyl-4-methyl-cumarin werden mit 6.18 g (0,022 mol) 3-(4-Benzamido-piperidino)-propylchlorid und 5.53 g Kalium-carbonat (0.04 mol) in 50 ml Dimethylformamid vereinigt. Nach vierstuendigem Ruehren bei 100 °C faellt nach dem Abkuehlen die gewuenschte Verbindung als Niederschlag aus, der aus Ethanol umkristallisiert werden kann.

Man erhaelt 5.4 g (58,6 % d. Th.) der Titelverbindung vom Schmp. 170-171 °C.

Aus dem eingeengten Filtrat erhaelt man durch Säulenchromatographie (Kieselgel/Methylenchlorid-Methanol = 9:1) weitere 2 g der reinen Verbindung.

Beispiel 14

In analoger Weise wie in Beispiel 13 beschrieben erhaelt man :

| | Bezeichnung | Ausbeute % | Schmelzpunkt °C (Lösungsmittel) |
|---|---|---|---|
| a) | 7-(3-[4-(4-Fluorbenzamido)-piperidino]-propoxy)-3-n-butyl-4-methyl-cumarin aus 7-Hydroxy-3-n-butyl-4-methyl-cumarin und 3-[4-(4-Fluorbenzamido)-piperidino]-propylchlorid | 53 | 185-186 (Ethanol) |
| b) | 7-[3-(4-Benzamido-piperidino)-propoxy]-4-methyl-8-n-propyl-cumarin aus 7-Hydroxy-4-methyl-8-n-propyl-cumarin und 3-(4-Benzamido-piperidino)-propylchlorid | 49 | 177-178 (Ethanol) |
| c) | 7-(3-[4-(4-Fluorbenzamido)-piperidino]-propoxy)-4-methyl-8-n-propyl-cumarin aus 7-Hydroxy-4-methyl-8-n-propyl-cumarin und 3-[4-(4-Fluorbenzamido)-piperidino]-propylchlorid | 68 | 176-177 (Ethanol) |
| d) | 7-[3-(4-Benzamido-piperidino)-propoxy]-3,4-dimethyl-8-n-propyl-cumarin aus 7-Hydroxy-3,4-dimethyl-8-n-propyl-cumarin und 3-(4-Benzamido-piperidino)-propylchlorid | 54 | 190-191 (Ethanol) |
| e) | 7-(3-[4-(4-Fluorbenzamido)-piperidino]-propoxy)-3,4-dimethyl-8-n-propyl-cumarin aus 7-Hydroxy-3,4-dimethyl-8-n-propyl-cumarin und 3-[4-(4-Fluorbenzamido)-piperidino]-propylchlorid | 51 | 206-207 (Ethanol) |
| f) | 7-[3-(4-Benzamido-piperidino)-propoxy]-3-n-butyl-4-methyl-8-n-propyl-cumarin aus 7-Hydroxy-3-n-butyl-4-methyl-8-n-propyl-cumarin und 3-(4-Benzamido-piperidino)-propylchlorid | 62 | 117-118 Säule $CH_2Cl_2$/ Methanol |
| g) | 7-(3-[4-(4-Fluorbenzamido)-piperidino]-propoxy)-3-n-butyl-4-methyl-8-n-propyl-cumarin aus 7-Hydroxy-3-n-butyl-4-methyl-8-n-propyl-cumarin und 3-[4-(4-Fluorbenzamido)-piperidino]-propylchlorid | 50 | 169-170 (Ethanol) |
| h) | 7-[3-(4-Benzamido-piperidino)-propoxy]-4,8-dimethyl-cumarin aus 7-Hydroxy-4,8-dimethyl-cumarin und 3-(4-Benzamido-piperidino)-propylchlorid | 63 | 183-184 (Ethanol) |
| i) | 7-[3-(4-Benzamido-piperidino)-propoxy]-3,4,8-trimethyl-cumarin aus 7-Hydroxy-3,4,8-trimethyl-cumarin und 3-(4-Benzamido-piperidino)-propylchlorid | 68 | 206 |
| j) | 7-[3-(4-Benzamido-piperidino)-propoxy]-4-methyl-8-acetyl-cumarin aus 7-Hydroxy-4-methyl-8-acetyl-cumarin und 3-(4-Benzamido-piperidino)-propylchlorid | 74 | 208-209 (Ethanol) |

Beispiel 14 (Fortsetzung)

| | Bezeichnung | Ausbeute % | Schmelzpunkt °C (Lösungsmittel) |
|---|---|---|---|
| k) | 7-[3-(4-Benzamido-piperidino)-propoxy]-3,4-dimethyl-8-acetyl-cumarin aus 7-Hydroxy-3,4-dimethyl-8-acetyl-cumarin und 3-(4-Benzamido-piperidino)-propylchlorid | 67 | 207-208 (Ethanol) |
| l) | 7-[3-(4-Benzamido-piperidino)-propoxy]-4-methyl-5-hydroxy-cumarin aus 5,7-Dihydroxy-4-methyl-cumarin und 3-(4-Benzamido-piperidino)-propylchlorid | 58 | amorph |
| m) | 7-[3-(4-Benzamido-piperidino)-propoxy]-4-methyl-5-methoxy-cumarin a) aus vorstehender Verbindung 14 1) durch Methylierung oder b) aus 5-Methoxy-7-hydroxy-4-methyl-cumarin und 3-(4-Benzamido-piperidino)-propylchlorid | 55 | amorph |
| n) | 7-(3-[4-(4-Fluorbenzamido)-piperidino]-propoxy)-4-methyl-5-hydroxy-cumarin aus 4-Methyl-5,7-dihydroxy-cumarin und 3-[4-(4-Fluorbenzamido)-piperidino]-propylchlorid | | |
| o) | 7-[3-(4-Benzamido-piperidino)-propoxy]-4,5-dimethyl-cumarin aus 4,5-Dimethyl-7-hydroxy-cumarin und 3-(4-Benzamido-piperidino)-propylchlorid | 88 | 175-176 |
| p) | 7-[3-(4-Benzamido-piperidino)-propoxy]-3,4,5-trimethyl-cumarin aus 3,4,5-Trimethyl-7-hydroxy-cumarin und 3-(4-Benzamido-piperidino)-propylchlorid | 61 | 193-194 $CH_2Cl_2$/Ether |

Beispiel 15

Es wurden Tabletten hergestellt : Jede Tablette enthält 10 mg 7-[3-(4-Benzamido-piperidino)-propoxy]-3,4-dimethyl-cumarin. Die Tabletten wurden gemäß der folgenden Rezeptur hergestellt :

| | |
|---|---|
| 7-[3-(4-Benzamido-piperidino)-propoxy]-3,4-dimethyl-cumarin | 10 g |
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,12 g verpreßt.

Die überlegene Wirkung der neuen Verbindungen wird gezeigt durch den Vergleich der inhibierenden Wirkung auf die passive kutane anaphylaktische Reaktion in Ratten, verursacht durch Injektion von Serum, das das Reagin für Ei-Albumin enthält. Diethylcarbamazin (1-Diethylcarbamoyl-4-methylpiperazin) wurde als Vergleichsverbindung benutzt. Im Einzelnen wurden die Tests wie folgt ausgeführt :

Serum, das Reagin (IgE-ähnlich) für Ei-Albumin enthielt, wurde erzeugt, indem man Ratten intramuskulär 0,1 ml einer Lösung des Antigens (10 mg/ml) in physiologischer Kochsalzlösung zusammen mit 0,5 ml Bordetella pertussis vaccine (Behring ; $2 \times 10^{10}$ Organismen/ml) injizierte. 9-14 Tage später wurden die Tiere über die Abdominal-Aorta ausgeblutet ; das Serum wurde gesammelt und bei

– 20 °C bis zum Verbrauch aufbewahrt. Der Titer des Serums, d.h. die höchste Verdünnung, die noch eine passive kutane anaphylaktische Reaktion (PCA) in Ratten nach einer 24-stündigen Latenzperiode verursachte, lag zwischen 1:8 und 1:32. Zur Verwendung in diesen Experimenten wurde das Serum 1:24 verdünnt. Die reaginartige Natur des Antikörpers wurde gezeigt durch seine Fähigkeit, PCA zu induzieren mit einer Latenzperiode von über 7 Tagen und außerdem durch Aufhebung der PCA-Aktivität durch 1-stündiges Erhitzen auf 56 °C.

Die Tiere wurden anästhesiert mit 2,2-Dichlor-1,1-difluoräthyl-methyl-äther, das unter dem Handelsnamen Penthran erhältlich ist, und wurden sensibilisiert durch Injektionen von 0,1 ml des Antiserums in die rasierten Abdominal-Flanken. Nach 48 Stunden für die reaginische PCA wurde den Tieren eine intravenöse Injektion von 1 ml einer Lösung von 0,5 Gewichtsprozent von Ei-Albumin und 0,25 Gewichtsprozent von Evans-Blau in physiologischer Kochsalzlösung gegeben.

Nach Abtöten und Ausbluten der Tiere, wurden die resultierenden blauen Flecke nach der Größe (in $mm^2$) und der Intensität (nach einer willkürlichen Punktwertung) ausgewertet. Das Produkt dieser Parameter wurde benutzt, um den Grad der Reaktion zu bestimmen und der Grad der Reaktion ohne aktives Material wurde als Standard benutzt, mit dem die Inhibierung der anaphylaktischen Reaktion in % ins Verhältnis gesetzt wurde.

6 Tiere wurden jeweils benutzt für die entsprechende Dosis und Kontrolle.

Das Testmaterial wurde p.o. 40 Minuten vor Gabe des Antigens verabreicht. Zu Vergleichszwecken wurde ebenfalls Diethyl-carbamoyl-4-methylpiperazin verwendet, das unter dem Handelsnamen Diethylcarbamazin erhältlich ist. Die Menge des Testmaterials wurde variiert, um die indizierte Dosis an aktivem Material zu bestimmen. In der folgenden Tabelle werden die Ergebnisse zusammengefaßt :

Homologe PCA-Reaktion in Ratten induziert durch Reagin

Ovalbumin (2 × kristallisiert) und Bord. pertussis ($2 \times 10^{10}$) ; Applikation der Verbindungen : p.o. 40 Minuten vor Ovalbumin, 6 Ratten/Dosis und 6 Kontrolltiere.

Tabelle

| Verbindungen | | Dosis (mg/kg) | % Inhibition von PCA |
|---|---|---|---|
| Diethylcarbamazin | | 100.0 | 47 |
| Beispiel | 1 | 3.0 | 50 |
| " | 2a | 3.0 | 59 |
| " | 2b | 3.0 | 36 |
| " | 2d | 3.0 | 52 |
| " | 4a | 3.0 | 61 |
| " | 6 | 3.0 | 30 |
| " | 7b | 3.0 | 23 |
| " | 8 | 3.0 | 34 |
| " | 10 | 3.0 | 54 |

**Ansprüche** (für die Vertragsstaaten : BE, CH, DE, FR, GB, IT, LU, NL, SE)

1. Arylether-Derivate der allgemeinen Formel I

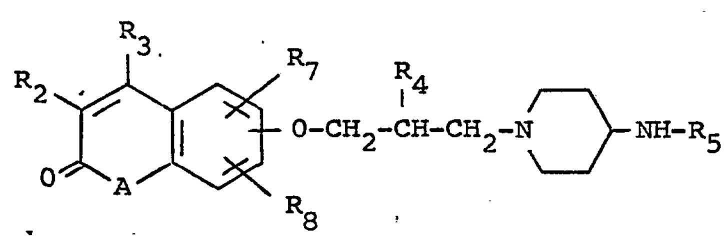

(I)

in der

A ein Sauerstoffatom oder die Gruppe N-$R_1$ darstellt, wobei

$R_1$ ein Wasserstoffatom oder ein niederer Alkylrest sein kann,

$R_2$ und $R_3$, die gleich oder verschieden sein können, jeweils ein Wasserstoffatom oder einen niederen Alkylrest,

$R_4$ ein Wasserstoffatom oder die Hydroxyl-Gruppe,

$R_5$ ein Wasserstoffatom oder eine Niederalkanoyl-Gruppe, die ein- oder mehrfach durch Halogen, durch Phenyl, oder durch Benzthiazol-Derivate substituiert sein kann, oder eine gegebenenfalls zweifach durch Methoxy substituierte Cinnamoyl-Gruppe, oder eine Benzoyl-Gruppe, die ein- oder mehrfach durch Halogen, Hydroxyl, Niederalkyl, Niederalkoxy, Carboxyl, Nitro, Amino, Nitril, Trifluormethyl, Carbamoyl oder Benzyl substituiert sein kann, oder eine Furancarbonyl-, Thiophencarbonyl-, oder Pyridincarbonyl-Gruppe, oder eine $C_3$-$C_7$-Cycloalkylcarbonyl-Gruppe oder eine Benzolsulfonyl- oder Methansulfonyl-Gruppe,

$R_7$ ein Wasserstoffatom, einen niederen Alkylrest oder einen niederen Alkanoylrest und

$R_8$ ein Wasserstoffatom, einen niederen Alkylrest, einen niederen Alkoxyrest oder die Hydroxylgruppe bedeuten, sowie deren pharmakologisch verträgliche Salze.

2. Arylether-Derivate gemäß Anspruch 1 wobei $R_2$, $R_3$, $R_4$, $R_5$, $R_7$ und $R_8$ die angegebene Bedeutung haben und A ein Sauerstoffatom darstellt.

3. Arylether-Derivate gemäß Anspruch 1, wobei A ein Sauerstoffatom, $R_2$ ein Wasserstoffatom, Methyl oder Butyl, $R_3$ ein Wasserstoffatom oder Methyl, $R_4$ ein Wasserstoffatom, $R_8$ ein Wasserstoffatom, Hydroxyl, Methyl oder Methoxy, $R_7$ ein Wasserstoffatom, Methyl, Propyl oder Acetyl darstellen und $R_5$ die angegebene Bedeutung hat.

4. Verfahren zur Herstellung von Verbindungen gemäß Ansprüche 1 bis 3 mit den hierin angegebenen Bedeutungen der Substituenten, dadurch gekennzeichnet, daß man eine Verbindung der allgemeinen Formel II

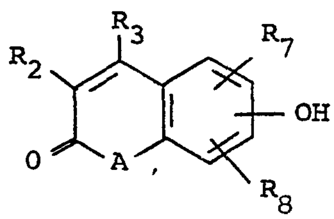

(II)

in der

A, $R_2$, $R_3$, $R_7$ und $R_8$ die oben angegebene Bedeutung haben, mit einer Verbindung der allgemeinen Formel III

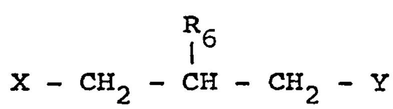

(III)

in der

X und Y reaktive Reste bedeuten und $R_6$ ein Wasserstoffatom, die Hydroxyl-Gruppe oder auch zusammen mit Y ein Sauerstoffatom sein kann, und einer Verbindung der allgemeiner Formel IV

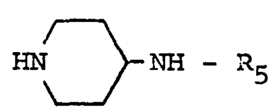

(IV)

in der

$R_5$ die obengenannte Bedeutung hat, umsetzt, anschließend gewünschtenfalls die Gruppe $R_5$ durch bekannte Verfahren in eine andere Gruppe $R_5$ umwandelt, für den Fall, daß $R_1$ ein Wasserstoffatom bedeutet, gegebenenfalls nachträglich N-alkyliert und das so erhaltene Reaktionsprodukt gewünschtenfalls in ein pharmakologisch verträgliches Salz überführt.

5. Arzneimittel, gekennzeichnet durch einen Gehalt an Verbindungen gemäß Ansprüche 1 bis 3 und übliche Träger- und Hilfsstoffen.

6. Verwendung von Verbindungen gemäß Ansprüche 1 bis 3 zur Herstellung von Arzneimitteln mit antiallergischen Eigenschaften.

7. Verbindungen gemäß Ansprüche 1 bis 3 zur Verwendung bei der Heilung von allergischen Krankheiten.

**Anspruch** (für den Vertragsstaat : AT)

Verfahren zur Herstellung von Verbindungen der allgemeinen Formel I

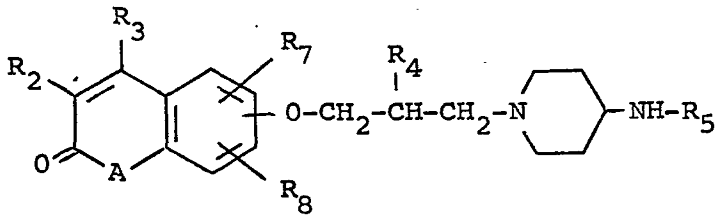 (I)

in der

A ein Sauerstoffatom oder die Gruppe N-$R_1$ darstellt, wobei

$R_1$, ein Wasserstoffatom oder ein niederer Alkylrest sein kann,

$R_2$ und $R_3$, die gleich oder verschieden sein können, jeweils, ein Wasserstoffatom oder einen niederen Alkylrest,

$R_4$ ein Wasserstoffatom oder die Hydroxyl-Gruppe,

$R_5$ ein Wasserstoffatom oder eine Niederalkanoyl-Gruppe, die ein- oder mehrfach durch Halogen, durch Phenyl, oder durch Benzthiazol-Derivate substituiert sein kann, oder eine gegebenenfalls zweifach durch Methoxy substituierte Cinnamoyl-Gruppe, oder eine Benzoyl-Gruppe, die ein- oder mehrfach durch Halogen, Hydroxyl, Niederalkyl, Niederalkoxy, Carboxyl, Nitro, Amino, Nitril, Trifluormethyl, Carbamoyl oder Benzyl substituiert sein kann, oder eine Furancarbonyl-, Thiophencarbonyl-, oder Pyridincarbonyl-Gruppe, oder eine $C_3$-$C_7$-Cycloalkylcarbonyl-Gruppe oder eine Benzolsulfonyl- oder Methansulfonyl-Gruppe,

$R_7$ ein Wasserstoffatom, einen niederen Alkylrest oder einen niederen Alkanoylrest und

$R_8$ ein Wasserstoffatom, einen niederen Alkylrest, einen niederen Alkoxyrest oder die Hydroxylgruppe bedeuten, sowie deren pharmakologisch verträgliche Salze, dadurch gekennzeichnet, daß man in an sich bekannter Weise eine Verbindung der allgemeinen Formel II

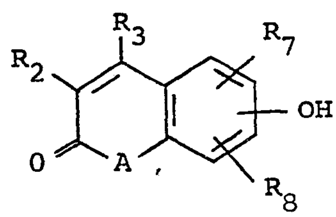 (II)

in der

A, $R_2$, $R_3$, $R_7$ und $R_8$ die oben angegebene Bedeutung haben, mit einer Verbindung der allgemeinen Formel III

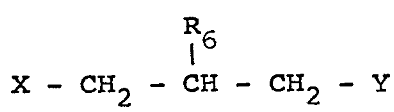, (III)

in der

X und Y reaktive Reste bedeuten und $R_6$ ein Wasserstoffatom, die Hydroxyl-Gruppe oder auch zusammen mit Y ein Sauerstoffatom sein kann, und einer Verbindung der allgemeinen Formel IV

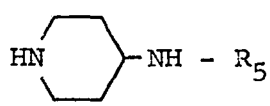 (IV)

in der

$R_5$ die obengenannte Bedeutung hat, umsetzt, anschließend gewünschtenfalls die Gruppe $R_5$ durch bekannte Verfahren in eine andere Gruppe $R_5$ umwandelt, für den Fall, daß $R_1$ ein Wasserstoffatom bedeutet, gegebenenfalls nachträglich N-alkyliert und das so erhaltene Reaktionsprodukt gewünschtenfalls in ein pharmakologisch verträgliches Salz überführt.

**Claims** (for the Contracting states : BE, CH, DE, FR, GB, IT, LU, NL, SE)

1. Aryl ether derivatives of the general formula I

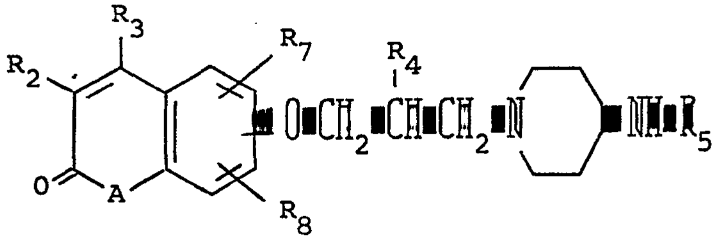

(I)

in which

A signifies an oxygen atom or the group N-$R_1$,

whereby

$R_1$ can be a hydrogen atom or a lower alkyl radical,

$R_2$ and $R_3$, which can be the same or different, each a hydrogen atom or a lower alkyl radical,

$R_4$ a hydrogen atom or the hydroxyl group,

$R_5$ a hydrogen atom or a lower alkanoyl group, which can be substituted one or more times by halogen, by phenyl or by benzthiazole derivatives ; or a cinnamoyl group optionally substituted twice by methoxy ; or a benzoyl group which can be substituted one or more times by halogen, hydroxyl, lower alkyl, lower alkoxy, carboxyl, nitro, amino, nitrile, trifluoromethyl, carbamoyl or benzyl ; or a furancarbonyl, thiophenecarbonyl or pyridine carbonyl group or a $C_3$-$C_7$-cycloalkylcarbonyl group or a benzenesulphonyl or methanesulphonyl group,

$R_7$ a hydrogen atom, a lower alkyl radical or a lower alkanoyl radical and

$R_8$ a hydrogen atom, a lower alkyl radical, a lower alkoxy radical or the hydroxyl group, as well as their pharmacologically acceptable salts.

2. Aryl ether derivatives according to claim 1, whereby $R_2$, $R_3$, $R_4$, $R_5$, $R_7$ and $R_8$ have the given meaning and A represents an oxygen atom.

3. Aryl ether derivatives according to claim 1, whereby A represents an oxygen atom, $R_2$ a hydrogen atom, methyl or butyl, $R_3$ a hydrogen atom or methyl, $R_4$ a hydrogen atom, $R_8$ a hydrogen atom, hydroxyl, methyl or methoxy, $R_7$ a hydrogen atom, methyl, propyl or acetyl and $R_5$ has the given meaning.

4. Process for the preparation of compounds according to claims 1 to 3 with the meanings of the substituents given herein, characterised in that one reacts a compound of the general formula II

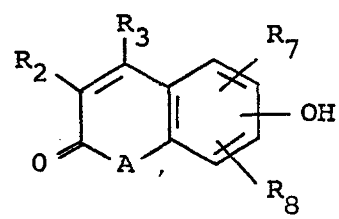

(II)

in which

A, $R_2$, $R_3$, $R_7$ and $R_8$ have the above-given meaning, with a compound of the general formula III

$$X - CH_2 - \overset{R_6}{\overset{|}{CH}} - CH_2 - Y \qquad \text{(III)}$$

in which

X and Y signify reactive residues and $R_6$ a hydrogen atom, the hydroxyl group or, together with Y, also an oxygen atom, and a compound of the general formula IV

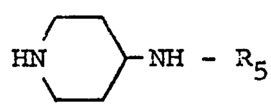

(IV)

in which

$R_5$ has the above-given meaning, subsequently, if desired, converts the group $R_5$ by known processes into another group $R_5$, for the case in which $R_1$ signifies a hydrogen atom, possibly subsequently N-alkylates and, if desired, converts the product thus obtained into a pharmacologically acceptable salt.

5. Medicaments, characterised by a content of compounds according to claims 1 to 3 and conventional carrier and adjuvant materials.

6. Use of compounds according to claims 1 to 3 for the preparation of medicaments with anti-allergic properties.

7. Compounds according to claims 1 to 3 for use in the healing of allergic diseases.

**Claim** (for the Contracting state : AT)

Process for the preparation of compounds of the general formula I

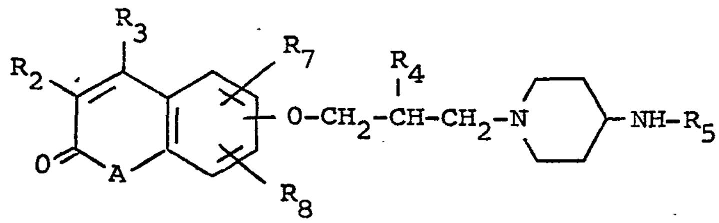

(I)

in which

A signifies an oxygen atom or the group N-$R_1$,

whereby

$R_1$ can be a hydrogen atom or a lower alkyl radical,

$R_2$ and $R_3$, which can be the same or different, each a hydrogen atom or a lower alkyl radical,

$R_4$ a hydrogen atom or the hydroxyl group,

$R_5$ a hydrogen atom or a lower alkanoyl group, which can be substituted one or more times by halogen, by phenyl or by benzthiazole derivatives ; or a cinnamoyl group optionally substituted twice by methoxy ; or a benzoyl group which can be substituted one or more times by halogen, hydroxyl, lower alkyl, lower alkoxy, carboxyl, nitro, amino, nitrile, trifluoromethyl, carbamoyl or benzyl ; or a furancarbonyl, thiophenecarbonyl or pyridine carbonyl group or a $C_3$-$C_7$-cycloalkylcarbonyl group or a benzenesulphonyl or methanesulphonyl group,

$R_7$ a hydrogen atom, a lower alkyl radical or a lower alkanoyl radical and

$R_8$ a hydrogen atom, a lower alkyl radical, a lower alkoxy radical or the hydroxyl group, as well as their pharmacologically acceptable salts characterised in that one reacts in per se known manner a compound of the general formula II

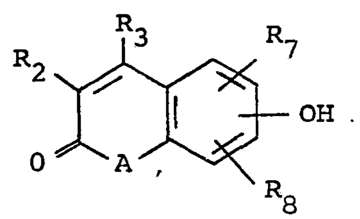

(II)

in which

A, $R_2$, $R_3$, $R_7$ and $R_8$ have the above-given meaning, with a compound of the general formula III

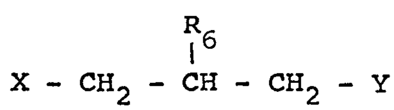

(III)

in which

X and Y signify reactive residues and $R_6$ a hydrogen atom, the hydroxyl group or, together with Y, also an oxygen atom, and a compound of the general formula IV

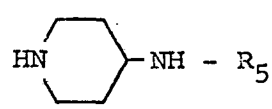

(IV)

in which

$R_5$ has the above-given meaning, subsequently, if desired, converts the group $R_5$ by known processes into another group $R_5$, for the case in which $R_1$ signifies a hydrogen atom, possibly subsequently N-alkylates and, if desired, converts the product thus obtained into a pharmacologically acceptable salt.

**Revendications** (pour les Etats contractants : BE, CH, DE, FR, GB, IT, LU, NL, SE)

1. Dérivés d'aryléthers de formule générale I

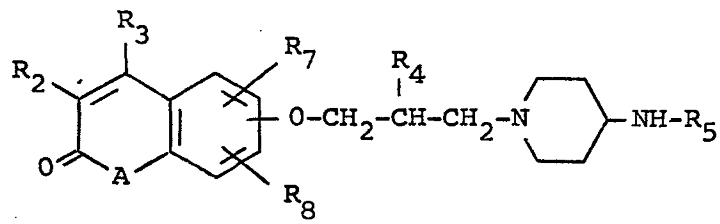

(I)

dans laquelle

A est un atome d'oxygène ou le groupe N-$R_1$ dans lequel

$R_1$ peut être un atome d'hydrogène ou un reste alkyle inférieur,

$R_2$ et $R_3$ pouvant être identiques ou différents, sont chacun un atome d'hydrogène ou un reste alkyle inférieur,

$R_4$ est un atome d'hydrogène ou le groupe hydroxyle,

$R_5$ est un atome d'hydrogène ou un groupe alcanoyle inférieur, pouvant être substitué une ou plusieurs fois par de l'halogène, du phényle ou par des dérivés de benzothiazole, ou un groupe cinnamoyle éventuellement substitué deux fois par méthoxy, ou un groupe benzoyle pouvant être substitué une ou plusieurs fois par de l'halogène ou par un groupe hydroxyle, alkyle inférieur, alcoxy inférieur, carboxyle, nitro, amino, nitrile, trifluorométhyle, carbamoyle ou benzyle, ou un groupe furannecarbonyle, thiophènecarbonyle ou pyridinecarbonyle, ou un groupe $C_3$-$C_7$-cyclo-alkylcarbonyle, ou un groupe benzènesulfonyle ou méthanesylfonyle,

$R_7$ est un atome d'hydrogène, un reste alkyle inférieur ou un reste alkanoyle inférieur et,

$R_8$ est un atome d'hydrogène, un reste alkyle inférieur, un reste alcoxy inférieur ou le groupe hydroxyle, ainsi que leurs sels pharmacologiquement tolérables.

2. Dérivés d'aryléthers selon la revendication 1, dans lesquels $R_2$, $R_3$, $R_4$, $R_5$, $R_7$ et $R_8$ ont la signification indiquée et A est un atome d'oxygène.

3. Dérivés d'aryléthers selon la revendication 1, dans lesquels A est un atome d'oxygène, $R_2$ est un atome d'hydrogène, un groupe méthyle ou butyle, $R_3$ est un atome d'hydrogène ou un groupe méthyle, $R_4$ est un atome d'hydrogène, $R_8$ est un atome d'hydrogène, un groupe hydroxyle, méthyle ou méthoxy, $R_7$ un atome d'hydrogène, un groupe méthyle, propyle ou acétyle et $R_5$ a la signification indiquée.

4. Procédé pour la fabrication de composés selon l'une quelconque des revendications 1 à 3 dans lesquels les substituants sont ceux ci-dessus indiqués, caractérisé en ce que l'on fait réagir un composé de formule générale II

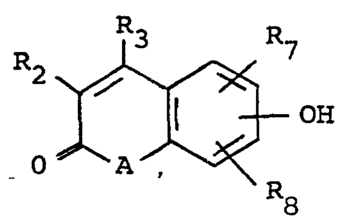

(II)

dans laquelle

A, $R_2$, $R_3$, $R_7$ et $R_8$ ont la signification ci-dessus indiquée, avec un composé de formule générale III

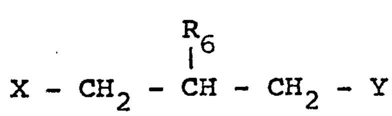

(III)

dans laquelle

X et Y sont des restes réactifs et $R_6$ est un atome d'hydrogène, le groupe hydroxyle, ou bien forme ensemble avec Y un atome d'oxygène et un composé de formule générale IV

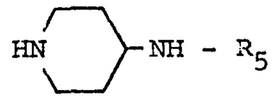

(IV)

dans laquelle

$R_5$ a la signification ci-dessus indiquée, et en ce qu'éventuellement ensuite on transforme le groupe $R_5$, selon un procédé connu en un autre groupe $R_5$, en ce que pour le cas où $R_1$ est un atome d'hydrogène on effectue éventuellement ultérieurement un N-alkylation, et en ce que l'on transforme le produit de réaction obtenu si l'on désire en un sel pharmacologiquement tolérable.

5. Médicament, caractérisé en ce qu'il comprend des composés selon l'une quelconque des revendications 1 à 3 et les substances de support et auxiliaires habituelles.

6. Utilisation des composés selon l'une quelconque des revendications 1 à 3 pour la fabrication de médicaments ayant des propriétés anti-allergiques.

7. Composés selon l'une quelconque des revendications 1 à 3 pour la thérapeutique des maladies allergiques.

**Revendication** (pour l'État contractant : AT)

Procédé pour la fabrication de composés de formule générale I

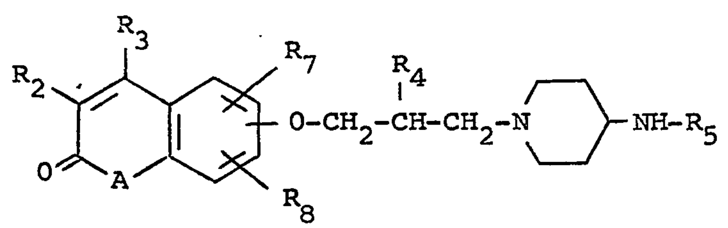

(I)

dans laquelle

A est un atome d'oxygène ou le groupe N-$R_1$ dans lequel

$R_1$ peut être un atome d'hydrogène ou un reste alkyle inférieur,

$R_2$ et $R_3$ pouvant être identiques ou différents, sont chacun un atome d'hydrogène ou un reste alkyle inférieur,

$R_4$ est un atome d'hydrogène ou le groupe hydroxyle,

$R_5$ est un atome d'hydrogène ou un groupe alcanoyle inférieur, pouvant être substitué une ou plusieurs fois par de l'halogène, du phényle ou par des dérivés de benzothiazole, ou un groupe cinnamoyle éventuellement substitué deux fois par méthoxy, ou un groupe benzoyle pouvant être substitué une ou plusieurs fois par de l'halogène ou par un groupe hydroxyle, alkyle inférieur, alcoxy inférieur, carboxyle, nitro, amino, nitrile, trifluorométhyle, carbamoyle ou benzyle, ou un groupe furannecarbonyle, thiophènecarbonyle ou pyridinecarbonyle, ou un groupe $C_3$-$C_7$-cyclo-alkylcarbonyle, ou un groupe benzènesulfonyle ou méthanesylfonyle,

$R_7$ est un atome d'hydrogène, un reste alkyle inférieur ou un reste alkanoyle inférieur et,

$R_8$ est un atome d'hydrogène, un reste alkyle inférieur, un reste alcoxy inférieur ou le groupe hydroxyle, ainsi que leurs sels pharmacologiquement tolérables, caractérisé en ce que l'on fait réagir un composé de formule générale II

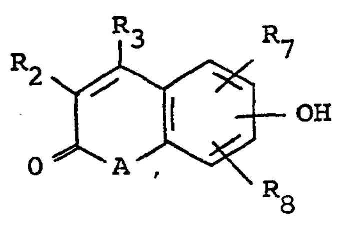

(II)

dans laquelle

A, $R_2$, $R_3$, $R_7$ et $R_8$ ont la signification ci-dessus indiquée, avec un composé de formule générale III

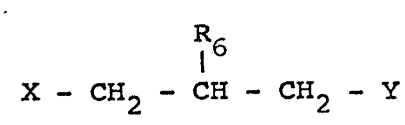

(III)

dans laquelle

X et Y sont des restes réactifs et $R_6$ est un atome d'hydrogène, le groupe hydroxyle, ou bien forme ensemble avec Y un atome d'oxygène, et un composé de formule générale IV

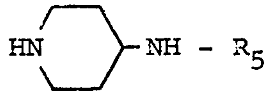

(IV)

dans laquelle

$R_5$ a la signification ci-dessus indiquée, et en ce qu'éventuellement ensuite on transforme le groupe $R_5$, selon un procédé connu en un autre groupe $R_5$, en ce que pour le cas où $R_1$ est un atome d'hydrogène on effectue éventuellement ultérieurement un N-alkylation, et en ce que l'on transforme le produit de réaction obtenu si l'on désire en un sel pharmacologiquement tolérable.